# EUROPÄISCHE PATENTANMELDUNG

(11) **EP 1 498 090 A1**
(43) Veröffentlichungstag der Anmeldung: **19.01.2005**
(21) Anmeldenummer: 03016157.4
(22) Anmeldetag: 16.07.2003
(51) Int. Cl.: A61F 2/36

(54) **Hüftprothese mit einem in den Oberschenkelknochen einzusetzenden Schaft**

(71) Anmelder: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll & Partner

(57) **Zusammenfassung**

Hüftprothese mit einem in eine Höhlung des Oberschenkelknochens zementfrei einzusetzenden Schaft (1). Der proximale Abschnitt des Schafts besteht aus einem Grundkörper, dessen Form der komplementären Form der mittels eines entsprechenden Werkzeugs im Knochen zu bildenden Höhlung entspricht. Auf dem Grundkörper erheben sich dorsal und ventral je eine Keilrippe (12), deren Breite im Mittel mindestens dreimal so groß wie ihre Höhe ist und deren Rückenfläche (13) abrasiv rauh ausgebildet ist.

## Beschreibung

Der dem Oberschenkel zugeordnete Teil einer Hüftprothese ist mit einem Schaft ausgestattet, der in eine Höhlung eingesetzt wird, die nach der Resektion des Hüftkopfs und Kopfhalses mittels eines geeigneten Werkzeugs im spongiösen, inneren Querschnittsbereich des Oberschenkelknochens geformt wird. Wenn der Schaft zementfrei eingesetzt wird, bemüht man sich, die Form der Höhlung möglichst weitgehend komplementär übereinstimmend mit der Form des Schafts auszubilden, damit die Prothese nach dem Einsetzen des Schafts sicher und fest sitzt. Dabei ist der proximale Schaftabschnitt, der im metaphysären Bereich des Knochens (etwa oberhalb des kleinen Trochanters) zu liegen kommt, so ausgebildet, daß er nicht nur vertikale Kräfte in Richtung des Oberschenkelknochens sondern auch quer dazu verlaufende und insbesondere nach medial gerichtete Kräfte übertragen kann. Damit auch die nach ventral und dorsal weisenden Oberflächenteile des proximalen Prothesenschafts an der Kraftübertragung auf den Knochen beteiligt werden, ist es bekannt, ihre Oberfläche so auszubilden, daß ein formflüssiger Verbund mit dem Knochengewebe zustande kommen kann. Dafür stehen zwei Möglichkeiten zur Verfügung, nämlich zum einen eine Mikro-Rauhigkeit der Oberfläche, die beispielsweise durch Glasstrahlen, poröse Beschichtung oder dergleichen zustande kommt und dem Knochen Gelegenheit gibt, in die Vertiefungen und Poren einzusprossen, und zum anderen Rippen, die von dem Schaftgrundkörper vorspringen. Diese beiden Möglichkeiten können auch gemeinsam angewendet werden. So ist es bekannt (EP-B-761 183) auf den dorsalen und ventralen Flächen des proximalen Schaftgrundkörpers Rippen aufzusetzen, die in Längsrichtung des Schafts verlaufen und deren Querschnitt sich von distal nach proximal keilförmig vergrößert. Derartige Rippen werden im folgenden als Keilrippen bezeichnet. Für die Herstellung der Höhlung wird eine Raspel verwendet, deren Gestalt dem Schaftgrundkörper ohne die Rippen entspricht. Beim Einsetzen des Schafts bildet der Schaftgrundkörper mit der Oberfläche der Höhlung einen Preßsitz. Die Keilrippen schneiden beim Einsetzen der Prothese in das spongiöse Knochengewebe ein. Aufgrund ihrer Keilform verdrängen und verdichten es. Dies trägt zum festen Sitz der Prothese bei. Dabei ergibt sich zum einen ein makroskopischer Formschluß zwischen dem Knochen und dem Prothesenquerschnitt dank der Rippe. Zum anderen ergibt sich nach dem Einwachsen von Knochengewebe in die rauhe oder porige Oberflächenstruktur ein mikroskopischer Formschluß. Diese beiden Wirkungen treten offensichtliche unabhängig voneinander ein und werden bei der Konstruktion einer Prothese ohne gegenseitige Rücksicht eingesetzt.

Die erwähnte Kompression des Knochenmaterials beim Einsetzen einer Keilrippe darf selbstverständlich nicht dazu führen, daß der Knochen gesprengt wird. Diese Gefahr ist um so größer, je breiter eine Rippe ist, weil sie dann der Knochenrinde eine um so größere Keilfläche zuwendet und eine um so größere Keilkraft gegen die Knochenrinde erzeugt. Bekannte Keilrippen sind deshalb schmal ausgeführt (EP-B-761 183, EP-B-159 462). Aus demselben Grunde bemüht man sich, die Keilwirkung der Rippen in die tangentiale Richtung zu lenken (DE-U-295 22 382, Blatt 4, Zeile 22). Zwar sind auch breite Rippen bei zementfrei einzusetzenden Hüftprothesen bekannt (EP-A-10 70 490). Dabei handelt es sich aber um Rippen, die zur Verdrängung und Kompression von Knochenmaterial aufgrund ihrer Form nicht geeignet sind, und deshalb eine solche Formung der Höhlung verlangen, daß ihr Volumen und ihre Form von vornherein in der Höhlung berücksichtigt wird. Bekannt sind ferner verhältnismäßig breite, von distal nach proximal sich verbreiternde Schaftvorsprünge an Prothesenschäften, die zur zementierten Implantation bestimmt sind und bei denen daher das zugehörige Werkzeug so ausgebildet ist, daß die künstliche Knochenhöhlung voluminöser ist als der Schaft einschließlich seiner rippenartigen Aufsätze.

Der Erfindung liegt die Aufgabe zugrunde, einen Prothesenschaft zur zementfreien Implantation mit Keilrippen und rauher Oberfläche zu schaffen, der eine rasche und großflächige Haftverbindung zwischen der Prothesenoberfläche und dem Knochengewebe ermöglicht. Die erfindungsgemäße Lösung besteht in den Merkmalen des Anspruchs 1, nämlich darin - kurz gesagt - daß die Keilrippe mit rauher Rückenfläche sehr breit ausgebildet ist. Sie ist im Mittel mindestens dreimal, vorzugsweise viermal, so breit wie hoch.

Nach bisherigen Erfahrungen muß man damit rechnen, daß eine derart breite Keilrippe ihre Keilwirkung hauptsächlich nach außen gegen die Knochenrinde entfaltet und dadurch die Gefahr einer Sprengung des Knochens heraufbeschworen wird. Diese Gefahr wäre in der Tat vorhanden, wenn die Rückenfläche der Rippe nicht rauh wäre. Die Rauhigkeit führt dazu, daß die Knochensubstanz, die in unmittelbaren Kontakt mit der Rippenrückenfläche kommt und der Keilwirkung und Relativbewegung ausgesetzt wird, zerrieben und zerkleinert wird und dadurch in einen fließfähigen Zustand gelangt, so daß sie zur Seite weg von der Rippenrückenfläche verdrängt werden und abfließen kann. Dies hat zwei Effekte. Zum einen ist die in Richtung normal zur Rippenrückenfläche erzeugte Kraft vergleichsweise gering, wodurch die möglicherweise schädigende Kraftausübung auf die Knochenrinde begrenzt wird. Zweitens befindet sich die Rippenrückenfläche im Endzustand der Implantation in unmittelbarer Nähe von ungeschädigter Knochensubstanz, in welcher das natürliche Gefäßsystem erhalten geblieben ist, da die zuvor zerriebene Knochensubstanz dank ihrer Fließfähigkeit sich entfernt. Daraus ergibt sich, daß der Vorgang des Einsprossens neuer Knochensubstanz die poröse Rückenfläche der Rippe sehr rasch nach der Implantation erreicht und daher schon nach kurzer Zeit großflächig ein inniger Oberflächenverbund geschaffen wird. Zwar mag es sein, daß auch bei den bisher bekannten Keilrippen mit rauher Oberfläche an deren Querschnittsspitze gleichfalls ein solcher Effekt auftrat. Da er aber auf eine sehr geringe Fläche beschränkt war, wurde er nicht wahrgenommen und fiel er auch nicht positiv ins Gewicht. An den Rippenflanken ist die Situation grundsätzlich anders. Bei einzeln stehenden Rippen ist in Kompressionsrichtung normal zur Flankenoberfläche ein verhältnismäßig weiter Kompressionsraum vorhanden, so daß die Knochensubstanz zwar komprimiert und teilweise gequetscht wird, aber die Pressung nicht so groß wird wie es für eine nennenswerte abrasive Wirkung der Rauhigkeit erforderlich wäre. Die Knochensubstanz bleibt mehr oder weniger an Ort und Stelle. Da die Gefäße darin größtenteils zerstört sind, bildet sie zunächst eine Sperre zwischen dem unverletzen Knochengewebe und der Prothesenoberfläche, die nach der Implantation von frischem Knochengewebe erst durchdrungen werden muß, bevor es in die rauhe Oberfläche der Prothese einsprossen kann und eine formflüssige Verbindung zustande kommen kann. Auch in den Zwischenräumen zwischen benachbarten Rippen ist die Situation anders als am Rippenrücken, weil dort die Kompression und Gewebezerstörung besonders ausgeprägt ist, ohne daß sich das zerstörte Gewebe entfernen kann. Auch in diesem Bereich kann also die Verbindung mit frischem Knochengewebe nur verzögert erfolgen.

Die Erfindung schafft somit den Vorteil, daß dank der Rauhheit des großflächigen Rippenrückens und der dadurch bewirkten Nähe zu unverletztem Knochengewebe sehr rasch eine feste Verbindung zwischen Prothesenoberfläche und Knochenoberfläche erfolgen kann.

Vorzugsweise ist die Rippe im gesamten proximalen Abschnitt mindestens dreimal, vorzugsweise viermal so breit wie hoch. Damit die abrasive Wirkung der Rauhigkeit bei der Relativbewegung der Rippenoberfläche gegenüber dem angepressten Knochengewebe stets hinreichend groß ist im Vergleich mit der durch Keilwirkung erzeugten Quetschung, soll der Keilwinkel, das heißt der Winkel zwischen der Fläche des Rippenrückens und der in lateral-medialer-Richtung verlaufenden Mittelebene (LM-Mittelebene) des Schafts, nicht größer als 5°, vorzugsweise nicht größer als 3,5° und weiter vorzugsweise nicht größer als 2,5° sein.

Auch die Breite der Rippe, das heißt ihre Abmessung in LM-Richtung, wächst vorzugsweise von distal nach proximal an, wobei der Winkel zwischen der lateralen Kante und der Längsrichtung des Schafts nicht größer sein sollte als 4°, vorzugsweise 3°. Dasselbe gilt für die laterale Kante.

Im Schaftquerschnitt soll die Rippenrückenfläche etwa parallel zu der LM-Mittelebene verlaufen. Der Winkel zwischen der Rippenrückenfläche und der LM-Mittelebene ist vorzugsweise nicht größer als 15°, wobei die Rippenhöhe zur lateralen Seite hin anwächst. Die mediale Flanke ist zweckmäßigerweise scharfkantig von der Rippenrückenfläche abgesetzt und verläuft im wesentlichen lotrecht zur LM-Ebene. Das selbe gilt zweckmäßigerweise auch für die laterale Kante, obwohl es dort weniger wichtig ist.

Damit die abrasive Wirkung der Rauhigkeit im Bereich des Rippenrückens hinreichend zur Wirkung kommt, soll die Rauhtiefe zwischen 0,05 und 0,5 mm liegen. In derselben Größenordnung liegt der Abstand benachbarter Rauhigkeitsspitzen.

Die Erfindung wird im folgenden näher unter Bezugnahme auf die Zeichnung erläutert, die ein vorteilhaftes Ausführungsbeispiel veranschaulicht. Es zeigen:

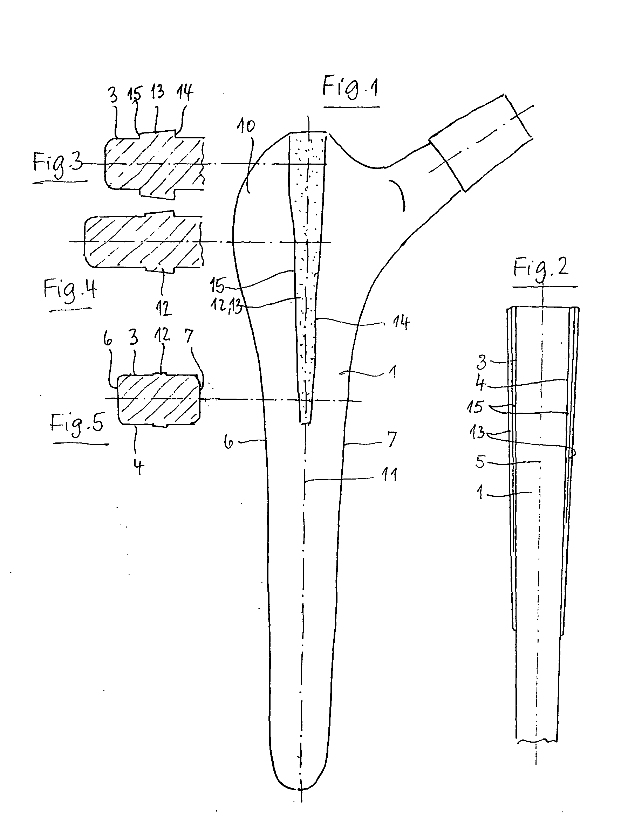

- Figur 1: eine Seitenansicht
- Figur 2: eine Ansicht von lateral und
- Figur 3 bis 5: Querschnitte durch den Prothesenschaft in jeweils entsprechender Höhe desselben.

Es handelt sich um eine Geradschaftprothese, das heißt der Schaft hat eine durchgehend gerade Längsachse und wird in gerader Richtung in das Femur implantiert. Der Schaft 1 umfaßt einen im Querschnitt im wesentlichen rechteckigen Grundkörper mit parallelen dorsalen und ventralen Seiten 3, 4, die sich nach distal keilförmig verjüngen. Sie schließen mit der lateral-medial verlaufenden Mittelebene 5 jeweils einen Winkel von weniger als 2° ein. Die lateralen und medialen Begrenzungsflächen 6, 7 des Schaftgrundkörpers verlaufen gleichfalls nach distal sich keilförmig verjüngend. Am proximalen Ende schließen sich der Prothesenhals mit einem Konus 9 zum Aufsetzen einer Gelenkkugel sowie im Bereich des großen Trochantas ein Lateralflügel 10 an.

Eine nicht dargestellte Raspel zum Formen des Knochenhohlraums, der den Prothesenschaft aufnehmen soll, hat die gleiche Gestalt wie der von den Flächen 3, 4 und 6, 7 begrenzte Grundkörper des Prothesenschafts, wie dies allgemein bekannt ist, um dem Prothesenschaft in der Knochenhöhlung nach der Implantation einen sicheren und festen Sitzt zu verleihen.

Auf die dorsalen und ventralen Flächen 3, 4 des Schaftgrundkörpers sind etwa mittig in Bezug auf die Schaftachse 11 Rippen 12 aufgesetzt, die die Form eines Keils mit geraden Begrenzungsflächen haben. Die Größe dieser Flächen ist die nach dorsal bzw. ventral gewendete Rückenfläche 13. Medial wird die Rippe durch eine etwa lotrecht zur Fläche 3 bzw. 4 verlaufende mediale Stirnfläche begrenzt. Dasselbe gilt für die laterale Stirnfläche 15.

Mit der Mittellinie 11 schließen die Stirnflächen 14, 15 ebenso wie die Begrenzungskanten der Rückenfläche 13 einen Winkel von jeweils etwa 2,5° ein. Der Winkel, den die Rippenrückenflächen 13 mit der LM-Mittelebene des Schafts einschließen, liegt bei 2°.

Die Rippenrückenfläche 13 verläuft etwa parallel zur LM-Mittelebene 5 des Schafts. Die Abweichung beläuft sich im dargestellten Beispiel auf weniger als 10°. An der medialen Kante ist die Rippe etwas höher als an der lateralen Kante, wodurch der makroskopische Formschluß zur Kraftübertragung von der Prothese nach medial auf den Knochen vergrößert wird.

Mindestens die Rückenfläche 13 der Rippe 12 ist durch Sandstrahlen, Plasmabeschichtung, Flammspritzen oder dergleichen mit einer rauhen und gegebenenfalls porösen Oberfläche versehen. Die Rauhigkeitserhebungen sind vorzugsweise scharfkantig geformt, so daß sie beim Einschieben des Schafts in den Knochen abrasiv auf die Knochensubstanz wirken. Eine solche Rauhigkeit kann auch an den übrigen Flächen des Prothesenschafts vorgesehen sein, um die innige Verbindung des Knochengewebes mit der Prothesenoberfläche zu ermöglichen. Für die Erfindung kommt es lediglich auf die Rauhigkeit der Rippenrückenfläche 13 an. Während der Schaftgrundkörper in der Knochenhöhlung komplementär vorgeformt ist und darin den gewünschten Presssitz ohne wesentlichen zusätzliche Verformung des Knochens findet, fehlt eine solche komplementäre Höhlungsform für die Rippen 12. Beim Eindringen des Schafts in den Knochen verdrängen sie eine ihrem Volumen entsprechende Menge des Knochengewebes. Wäre die Rückenfläche glatt, würde die laminare Struktur des Knochens dabei lediglich zusammengepresst und verdichtet werden, wobei der flüssige Inhalt der lamellaren Zwischenräume entweichen würde. Dank der Abrasivität des Rippenrückens werden im Falle der Erfindung die Knochenlamellen jedoch zerrieben und zerschnitten. Dadurch können sie mit dem flüssigen Zwischenrauminhalt aus dem Bereich zwischen der Rippenrückfläche und dem hinter ihnen liegenden festen und unverletzten Knochengeweben entweichen. Zum einen wird dadurch die Pressung herabgesetzt, die aufgrund der Keilwirkung der Rippen zwischen diesen und dem Knochen entsteht. Zum anderen wird die spongiöse Knochensubstanz, die sich zwischen der Rippenrückenfläche und der harten Knochenrinde befindet, nicht in ihrer Gesamtheit zusammengepresst und geschädigt. Vielmehr bleibt sie bis nahe an die Rippenoberfläche heran gesund und kann daher durch rasch zur Schaftoberfläche vordringendes und in deren Rauhigkeit einsprossendes, frisches Knochengewebe zur raschen Prothesenfestigung beitragen.

## Patentansprüche

1. Hüftprothese mit einem in eine Höhlung des Oberschenkelknochens zementfrei einzusetzenden Schaft (1), dessen proximaler Abschnitt aus einem Grundkörper und je einer dorsal und ventral davon vorspringenden Rippe (12) besteht, die parallel zur Schaftrichtung (11) verläuft, im Querschnitt von distal nach proximal zunimmt und eine rauhe Oberfläche aufweist, sowie Werkzeug, das zum Formen der mit der Form des Grundkörpers im wesentlichen übereinstimmenden Höhlung ausgebildet ist, **dadurch gekennzeichnet, daß** die Rippe im Mittel mindestens dreimal so breit wie hoch ausgebildet ist.

2. Hüftprothese nach Anspruch 1, **dadurch gekennzeichnet, daß** sie eine Geradschaftprothese ist.

3. Hüftprothese nach Anspruch 1 oder 2, **dadurch gekennzeichnet, daß** die Rückenfläche (13) der Rippen (12) im Schaftquerschnitt etwa parallel zur LM-Mittelebene (5) verläuft.

4. Hüftprothese nach Anspruch 3, **dadurch gekennzeichnet, daß** die Höhe der Rippe an ihrer medialen Kante (14) größer ist als an ihrer lateralen Begrenzung (15).

5. Hüftprothese nach einem der Ansprüche 1 bis 4, **dadurch gekennzeichnet, daß** die mediale Stirnfläche (14) der Rippe etwa lotrecht zur Oberfläche (3, 4) des Schaftgrundkörpers verläuft.

6. Hüftprothese nach einem der Ansprüche 1 bis 5, **dadurch gekennzeichnet, daß** die Rauhtiefe der Rippenrückenfläche (13) zwischen 0,05 und 0,5 mm liegt.

7. Hüftprothese nach Anspruch 6, **dadurch gekennzeichnet, daß** der Spitzenabstand der die Rauhigkeit bildenden Erhebungen in derselben Größenordnung wie die Rauhtiefe liegt.

8. Hüftprothese nach einem der Ansprüche 1 bis 7, **dadurch gekennzeichnet, daß** die Rauhigkeit der Rippenrückenfläche (13) in Bezug auf Knochengewebe abrasiv ist.
